# EUROPEAN PATENT SPECIFICATION

(11) **EP 3 975 897 B1**
(45) Date of publication and mention of the grant of the patent: **17.12.2025**
(21) Application number: 19755523.8
(22) Date of filing: 27.05.2019
(51) Int. Cl.: A61B 18/14, A61B 18/00

(54) **BENDABLE ELECTROSURGICAL ELECTRODE, BENDING TOOL AND METHODS**
BIEGBARE ELEKTROCHIRURGISCHE ELEKTRODE, BIEGEWERKZEUG UND VERFAHREN
ÉLECTRODE ÉLECTROCHIRURGICALE PLIABLE, OUTIL DE PLIAGE ET PROCÉDÉS

(30) Priority: 25.05.2018 GB 201808673
(43) Date of publication of application: 06.04.2022
(73) Proprietor: Swiss Medical Instruments AG, 8832 Wollerau (CH)
(72) Inventor: GASSER, André, 8832 Wollerau (CH)
(74) Representative: Körner, Thomas Ottmar
(86) International application number: PCT/EP2019/063702
(87) International publication number: WO 2020/239199

(56) References cited:
- WO-A1-96/39954
- US-A- 5 487 757
- US-A1- 2015 245 868

## Description

### Field of the invention

The invention relates to the field of surgical instruments such as can be used for ablation of tissue in arthroscopy, for example, which comprise a probe or wand which can be bent by a surgeon to suit the requirements of a particular operation. In particular, but not exclusively, the invention relates to bendable electrosurgical electrodes.

### Background of the invention

Keyhole surgery such as arthroscopy may be carried out with long, narrow surgical instruments inserted through a small incision, usually guided by camera or external imaging. It requires instruments which can be inserted into the patient's body and reach a target operation site some distance from the entry incision. The target may be inaccessible to rigid, straight instruments, for example due to the geometry of the intervening bone and tissue. In this description, the particular example of electrosurgical ablation in a distracted hip joint, where the ablation electrode must be manipulated in the space between between the femoral head and the acetabulum, will be used to illustrate the principles of the invention. However, the invention may also find beneficial application in other surgical applications in which a long, narrow probe is required to be bent to a particular shape or angle.

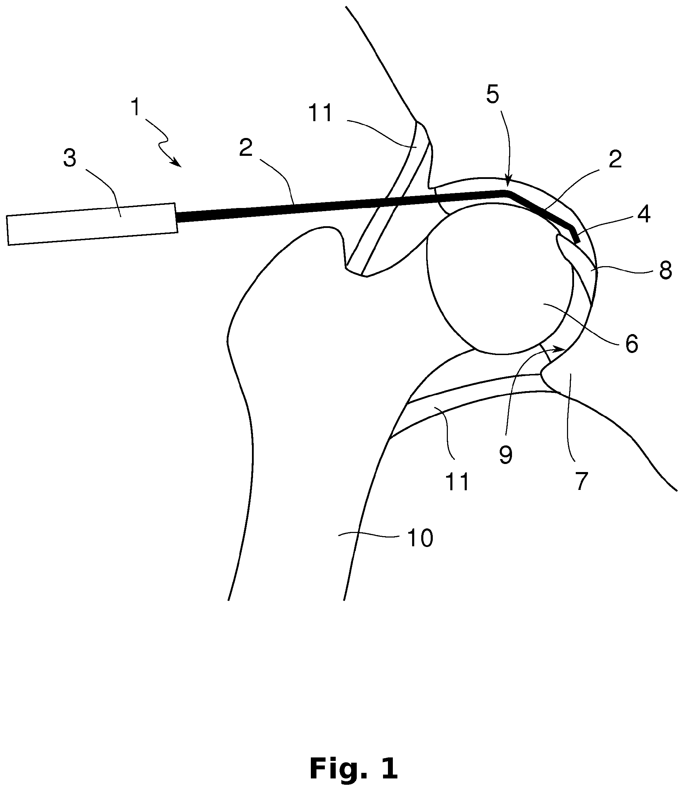

As mentioned above, electrosurgical electrodes may be used, for example, to ablate tissue in regions which are inaccessible along a straight path. An example is shown in figure 1, which shows a distracted hip joint, and an electrode 1, with its probe 2 inserted through the capsule 11 to reach the foveal ligament 8 which connects the head 6 of the femur 10 to the acetabulum 7. The electrode is guided by the surgeon, using the handle 3, to bring the electrode tip 4 of the probe 2 to the target site. In this example, the probe 2 is of the fixed-bend type and has a pre-bent probe 2 with a fixed bend 5. A similar arrangement, with a different probe geometry, can be used to ablate a region of the labrum 9. The probe 2 (also referred to as wand or shaft) of such electrodes 1 may be a disposable part, designed for one-off use. For electrosurgery such as ablation, the electrode 1 may be supplied with electrical power via cables (not shown) through the handle 3.

### Prior art

It is known to provide pre-bent electrosurgical electrodes for particular applications. These are electrodes which are manufactured and supplied already bent with a predetermined bend amount. They have the advantage that they can be fabricated simply and with great flexural strength, so that they can be used in surgical situations where it is important that the electrode does not bend. As an example, a surgeon may attempt to rotate the bent part of the probe past an immovable obstruction; the rotation may require significant force, and fixed-bend electrodes are fabricated such that they do not bend when subjected to such forces. As described above with reference to such figure 1, such a situation may arise during a hip arthroscopy, when the surgeon tries to rotate the electrode around the femoral head.

Fixed-bend electrodes have the disadvantage that the surgeon must keep a stock of multiple electrodes with different bend profiles and tip angles. In order to overcome this problem, it has also been suggested to provide electrodes which can be custom-bent for particular applications. International application WO2004/050171 describes an electrode which can be bent in a single plane. However, the fabrication of such an electrode is complicated. Furthermore, its structure has by design a reduced flexural strength, which means that the electrode, once bent, lacks sufficient rigidity in the bending plane to be used in applications where a strong, rigid electrode is required, such as the femoral head situation described above. The rotation of the probe of WO2004/050171 against the femoral head (which may be carried out with significant manual force) may cause the probe to deform in the bending plane. As a result, the surgeon, if he or she realises the problem, may have to withdraw the bendable electrode and attempt the operation again using a fixed-bend electrode instead. Or, if the surgeon is unaware of the problem, he or she may attempt to perform the ablation using an electrode probe which does not have the intended bend geometry, which may result in an incorrect ablation of the target site.

It is known from patent applications US20150245868 and US20160367313 to provide an electrode having a wand which can be bent at the time of surgery, and which can be re-bent during surgery to suit the requirements of the particular surgical application, such as tonsillectomy. The solution proposed in these documents is to fabricate the electrode wand with a section which is deliberately weakened, for example by the inclusion of notches in the wall of the probe, or by softening of the material of the probe, so that the section can be repeatedly bent. In order for the electrode to be repeatedly bendable, the section must retain its reduced flexural strength during and after bending, thereby rendering the electrode unsuitable for applications, such as the ablation of the labrum described above, where a strong, rigid electrode is required. US20150245868 and US20160367313 also describe a bending tool which can be used to apply the bending moment required to bend the weakened section. The bending tool fits on to the probe with its handle parallel to the probe. This 'end-on' collinear arrangement of the known bending tool is apparently sufficient to enable a surgeon to bend the prior art probe, because of the latter's reduced flexural strength, but it has the disadvantage that, if used to bend a probe with greater flexural strength, the 'end-on' bending tool would not be sufficient on its own, ie purely as a hand-held device. The surgeon would be obliged to use the tool to press the handle-end of the electrode against a fixed surface, which may damage the electrode.

There is thus a need for an electrosurgical device having a probe which has a flexural strength comparable to that of fixed-bend electrodes, but which can nevertheless be bent by hand to a degree specified by the surgeon.

### Brief description of the invention

The present invention aims to overcome at least some of the above disadvantages of prior art surgical instruments. To this end, an electrosurgical device according to the invention is described in the attached claim 1, methods according to the invention are described in claims 9 and 11, and a bending tool according to the invention is described in claim 12. Further variants of the invention are described in the dependent claims and in the following description.

The combination of the novel features in each of the independent claims enables the electrosurgical device to be used in situations where a) a customised bend geometry is required, and b) significant force, in particular rotational force, may be exerted on the probe by the surgeon during the operation.

The invention will be described in detail with reference to the attached drawings, in which:
Figure 1 shows a distracted hip joint and a prior art surgical instrument.
Figure 2 shows in schematic cross-sectional view an example of an electrosurgical device according to the invention.
Figures 2b and 2c show cross-sectional views of the device of figure 2a in planes A-A and B-B respectively.
Figures 3a to 3d show lateral elevation, plan, end elevation and isometric projection views respectively of a bending tool according to the invention.
Figure 4 shows in schematic plan view the bending tool of figures 3a to 3d used for bending the electrosurgical device of figure 2a.
Figure 5 shows in isometric projection view a variant of the bending tool of figures 3a to 3d according to the invention.
Figure 6 shows in schematic plan view the electrosurgical device and bending tool of figure 4 after bending.
Figure 7 shows the electrosurgical device of figure 6.
Figure 8 shows the bending tool of figure 6.

It should be noted that the figures are provided merely as an aid to understanding the principles underlying the invention, and should not be taken as limiting the scope of protection sought. Where the same reference numbers are used in different figures, these are intended to indicate similar or equivalent features. It should not be assumed, however, that the use of different reference numbers is intended to indicate any particular degree of difference between the features to which they refer.

### Detailed description of the invention

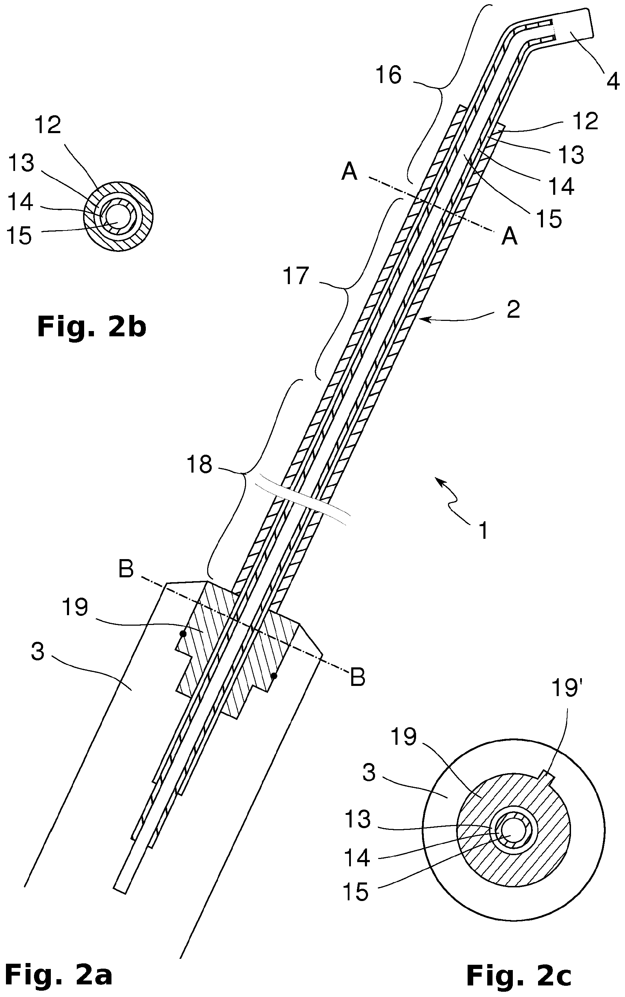

As described above in relation to figure 1, an electrosurgical device 1 may need to be bent in order for its tip 4 to reach the target site for electrosurgery (eg ablation of tissue). An example of an unbent electrode 1 is shown in figures 2a to 2c. The device comprises a handle 3 and a probe 2 with a tip 4 at distal portion 16 of the probe 2, bendable medial portion 17 of the probe 2 and proximal portion 18 of the probe 2. In this example, the electrode is a bipolar device, and the probe 2 has two electrical conductors 13 and 15, separated by a first insulator 14 and surrounded by a second insulator 12. The invention may also be implemented in monopolar electrodes or other probe-like surgical instruments. The conductors 13 and 15 of the example bipolar electrode supply electrical energy to the tip 4 in known fashion. The electrode may include aspiration and/or irrigation channels (not shown), for example passing through the inner electrical conductor 13. As shown in figures 2b and 2c, the conductor and insulator parts of the probe 2 preferably have a substantially circular cross-section. The handle 3 of the device 1 is advantageously detachable from the probe 2. To this end, the probe 2 comprises engagement part 19 for detachably fixing to the handle 3. Electrical power to the probe's connectors 13 and 15 may be provided via connectors (not shown) in the handle. The engagement part 19 preferably comprises a rotation-blocking engagement means 19', which prevents the handle 3 from rotating relative to the probe 2 when the device 1 is being manipulated by a surgeon. For the sake of clarity, the device of figure 2a is shown with the proximal portion of the probe foreshortened.

As will be described below, the medial portion 17 of the probe 2 is manufactured to be bendable by hand, but to have greater flexural strength after bending. To this end, at least the outer conductor 15 of the probe 2 is manufactured from such a material, and is treated in such a way, that its flexural strength is significantly greater (for example 20% or more) after bending than before bending. As mentioned above, it is known in the prior art to provide a probe which has a sufficiently low flexural strength to be bent by hand. It is also known to use a bending tool which fits on to the probe end-on, with its handle parallel to the probe. However, it is an aim of the invention to provide a bent probe whose bent portion has a flexural strength which is greater than, or almost as great as, that of the unbent portions, and the prior art bending tool would not be suitable for bending such a probe, for reasons explained above.

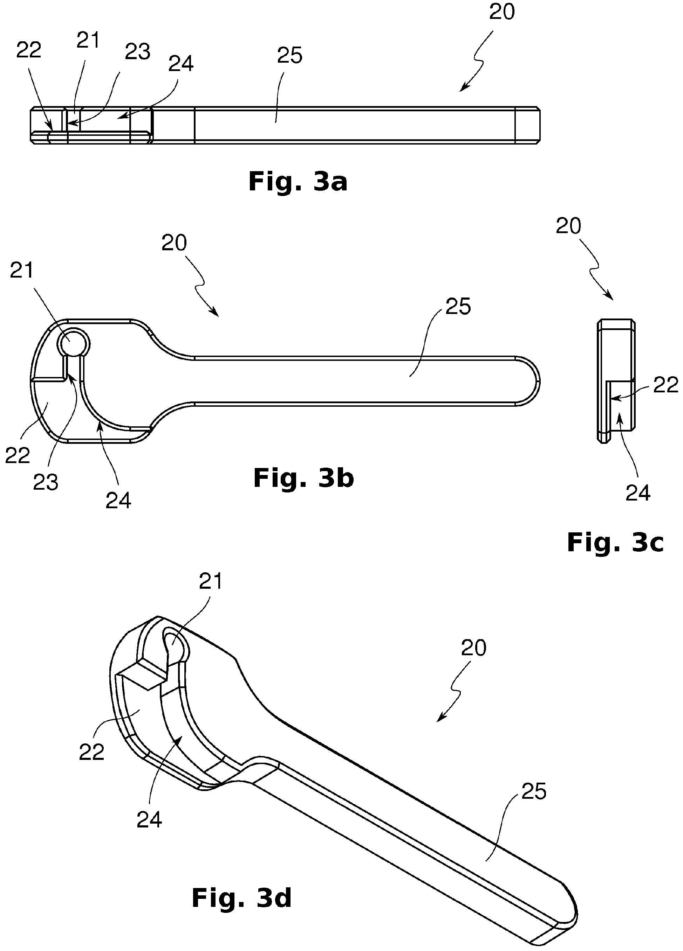

Figures 3a to 3d show a bending tool 20 which is designed to permit the user (eg the surgeon) to easily hold the electrode in one hand and the bending tool in the other, while enabling him or her to exert significant bending moment on the electrode probe by bringing his or her hands towards each other. It comprises a substantially straight, elongate handle 25 and a receiving region 21 for receiving the tip 4 and the distal portion 16 of the probe 2. The receiving region is shown as a closed region, such that the linear position of the probe 2 to be bent is defined. Alternatively, the receiving region may be open, such that the probe 2 may be positioned at any linear position relative to the bending surface, so that the user can determine where on the probe shaft the bend is made. Retaining and guiding surfaces 22 and 23 retain and guide the probe 2 while it is subjected to the bending moment. Bending surface 24 defines the geometry of the bent probe. The amount of bending may be determined by the surgeon. The bending surface 24 may have a constant radius of curvature, as shown, or it may have a radius of curvature which varies along its peripheral length. The bending surface 24 may preferably have a constant radius of curvature of between 10mm and 60mm, or more preferably between 20mm and 50mm.I In the case of a variable curvature, the bending surface may preferably have a minimum radius of curvature which is between 10mm and 60mm, or more preferably between 20mm and 50mm.

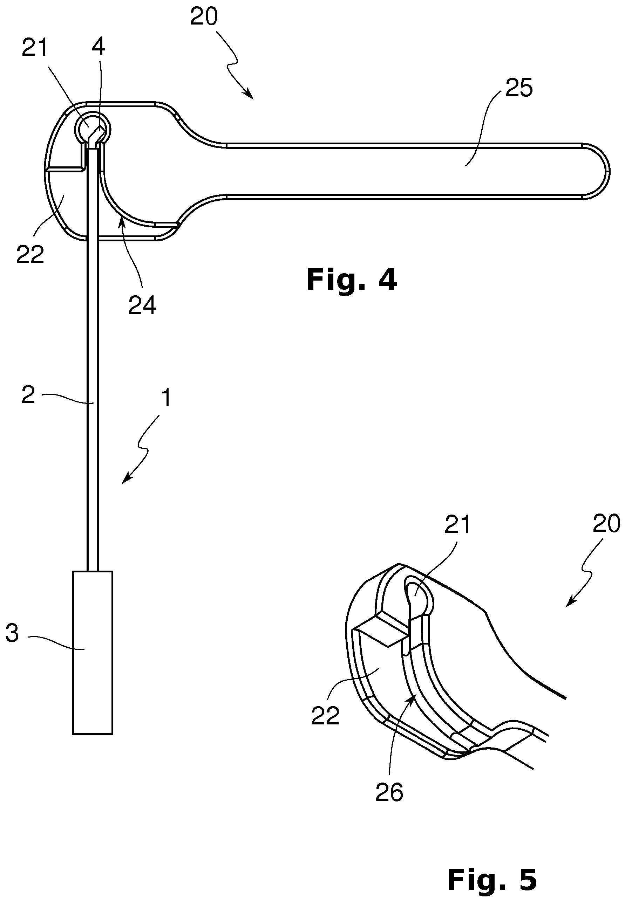

Figure 4 shows a surgical instrument such as the one illustrated in figure 2a engaged in the bending tool of figures 3a to 3d. The tip portion 4 is placed in the receiving region (recess) 21 and the probe 2 can then be bent by holding the handle 3 of the electrode 1 in one hand, and the handle 25 of the bending tool 25 in the other, and exerting a manual force to bring the two handles 3, 25 towards each other, thereby bending the probe 2 around the bending surface 24. The mutual angle of the two handles (90° as illustrated) allows the user to exert considerable controlled force during the bending. If further bending is required after the user has bent the probe, the force required will be even greater, and the reduced angle enables the user to exert the increased force.

Figure 5 shows a variant of the bending tool 20 in which the bending surface 24 is provided with a recessed profiled channel 26 which corresponds to the shape of the probe 2. The profiled channel 26 helps to avoid a flattening or crimping of the probe's tubular components 13, 15 during bending.

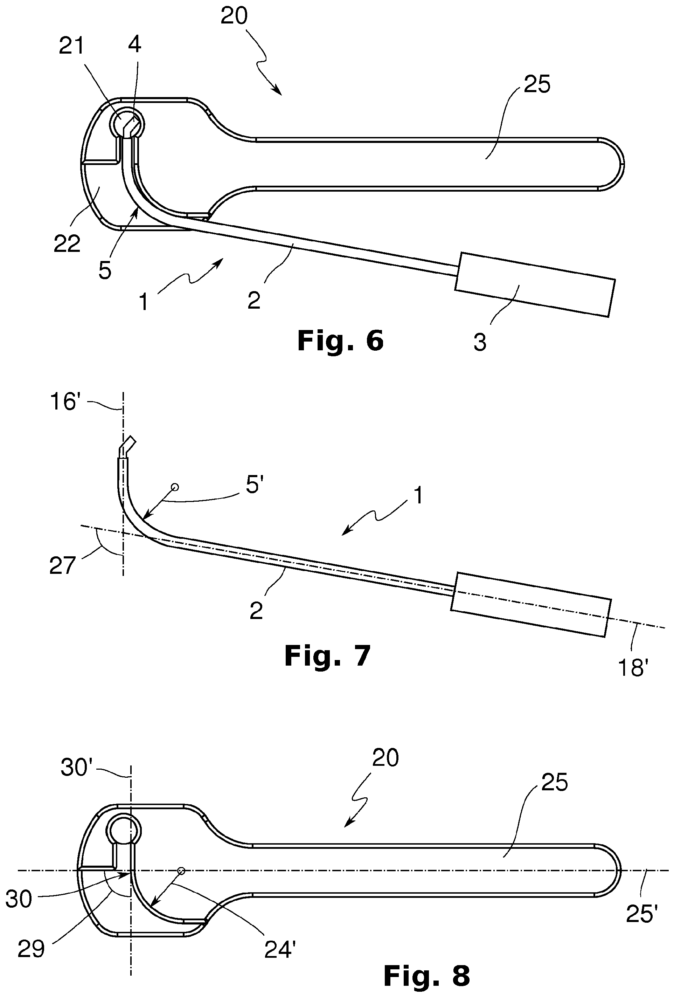

Figure 6 shows the bending tool 20 in use to form a bend 5 in the probe 2 of an electrode 1 such as the one described above. As shown in figure 7, the resulting bent probe 2 has a deviation angle 27 of eg more than 20° (80° as illustrated), between the axes 16' and 18' of the proximal 18 and distal 16 portions of the probe respectively. The bending radius 5' of the bend 5 may preferably be between 10mm and 60mm, or more preferably between 20mm and 50mm, for example.

Figure 8 shows the angle 29 at which the bending is started. This is the angle between the longitudinal axis 25' of the handle 25 and the axis 30', which is the tangent of the initial contact part 30 of the curved bending surface 24. The angle 29 may preferably by less than 135°, and more preferably less than 110°, in order that the user may exert enough bending force by bringing his/her hands together. In the illustrated example, the angle 29 is shown as 90°. Figure 8 also shows the radius of curvature 24' of the bending surface 24. As mentioned above, this radius preferably has a minimum value which is between 20mm and 50mm.

As mentioned above, the probe is designed to have a low enough flexural strength to be bendable by hand in its straight state, and a high enough flexural strength to resist bending when in use in its bent state. This can be achieved by judicious selection of materials for the principle elements which contribute to the flexural strength of the probe, and by suitable treatment to reduce the flexural strength without significantly reducing the strain hardening exponent of the material. In the case of the bipolar electrode described above, such a principle element may be the outer conducting tube 13, for example. The material may preferably be stainless steel with a strain hardening exponent of at least 0.4. Treatment preferably comprises subcritical or intercritical annealing at a temperature between 500°C and 700°C, or soft annealing at a temperature of between 700° and 900°. The material and the treatment are preferably such that the material has a flexural strength after treatment which is at least 80% but less than 95% of the flexural strength of the material before treatment, and a flexural strength after bending through at least 20° using the example bending tool 20, which is at least 85% of the flexural strength of the material before treatment. In the example embodiment, the flexural strength of the untreated tube material (stainless steel) may preferably be more than 200 MPa, and that of the treated tube 180 MPa or more. The stainless steel is selected such that the strain hardening which results from bending the tube 13 to a deviation angle of 20° or more increases its flexural strength to 190 MPa or more. The flexural strength may preferably be determined using the three-point flexural bending test, with the distal and proximal portions 16, 18 of the probe supported on the outer points and the bending force applied to the medial portion 17.

The example embodiment described above is intended to be illustrative and not limiting.

## Claims

1. An electrosurgical device (1) comprising;
a bendable elongate wand member (2);
a handle (3) at a proximal end of the wand member, the handle (3) being detachable from the wand member (2);
an operational tip portion (4) at a distal end of the wand member (2);
the wand member (2) having a first, proximal portion (18) for remaining straight when the wand member is bent, a second, distal portion (16) comprising the operational tip portion (4), and a third, bendable portion (17), located between the first and second portions (16, 18) for being plastically deformed by manual bending with a bend radius of between 10mm and 50mm, or more preferably between 20mm and 50mm so as to provide a deviation angle (27) between the first and the third portions of at least 20 degrees;
the first portion having a first flexural strength and the third portion having a second flexural strength, lower than the first flexural strength;
wherein
the wand member comprises a tubular element extending substantially along the length of the first, second and third length portions and made of a metal which, in the second length portion, has a strain hardening exponent greater than 0.4 in its unbent state;
**characterised in that**
the device comprises blocking means (19') for preventing rotation of the tubular element, relative to the handle, about a longitudinal axis of the wand member;
wherein the second flexural strength is at least 80% of the first flexural strength when the device is in an unbent configuration, before the said bending; and the second flexural strength is at least 85% of the first flexural strength when the device is in a bent configuration, after the said bending.

2. A device according to claim 1, wherein:
the tubular element (13) is made of a metal having a strength coefficient greater than 1200 MPa.

3. Device according to one of claims 1 or 2, wherein the first flexural strength is greater than 200 MPa and the second flexural strength is greater than 180 MPa.

4. Device according to one of the preceding claims, wherein the metal is stainless steel.

5. Device according to one of the preceding claims, wherein the first length portion is at least 80mm long and the second length portion is at least 25mm long.

6. Device according to one of the preceding claims, where the wand member (2) is deformable such that a distance to a midpoint of the said bending (5) from the distal end of the wand member, measured along the tubular element (13), is less than 50mm.

7. Device according to one of the preceding claims, wherein the electrosurgical device (1) is a monopolar electrosurgical electrode.

8. Device according to one of claims 1 to 6, wherein the electrosurgical device (1) is a bipolar electrosurgical electrode.

9. A method of manufacturing a device according to one of claims 1 to 8,
**characterised by** a processing step of annealing or normalising the metal of the second length portion such that:
- the second flexural strength before the bending is greater than 80% of the first flexural strength;
- the second flexural strength after the bending is greater than 90% of the first flexural strength.

10. Method according to claim 9, wherein the processing step comprises subcritical or intercritical annealing at a temperature between 500 and 700 degrees, or soft-annealing at a temperature of between 700 and 900 degrees.

11. Method of bending the wand member (2) of an electrosurgical device (1) according to one of claims 1 to 8, the method comprising:
- providing a bending tool (20) comprising a recess (21) for securing the tip portion (4) in anchored engagement during the bending, the bending tool further comprising a curved bending surface (24) having a minimum curvature radius of between 20mm and 50mm, and an elongate handle portion (25) having a longitudinal axis (25');
- engaging the tip portion (4) in said anchored engagement with the said recess (21);
- manually applying a force between the handle portion (3) and/or length portion of the electrosurgical device with one hand and the handle (25) of the bending tool (20) with the other hand so as to bend the second length portion (17) around the bending surface (24);
**characterised in that,** at the start of the bending, the longitudinal axes (16', 25') of the wand member and the handle (25) of the bending tool are at an angle of less than 135 degrees to each other.

12. Bending tool (20) for bending the wand (2) of a device (1) according to one of claims 1 to 8 in a method according to claim 11, the bending tool comprising:
- an elongate handle (25) having a longitudinal axis;
- a curved bending surface (24) having a minimum radius of curvature between 10mm and 60mm, or more preferably between 20mm and 50mm;
- engagement means (21) for retaining the tip portion (4) of the wand member (2) in anchored engagement during bending whereby, when the tip portion (4) is engaged with the engagement means (21) in initial engagement before the start of bending, the second length portion (17) of the wand member can rest in contact with an initial contact part (30) of the curved bending surface (24), with the longitudinal axis (18') of the wand parallel to a tangent (30') to the initial contact part (30) of the curved bending surface (24);
wherein the said tangent (30') is at a predetermined angle (29) to the longitudinal axis of the handle of the bending tool;
**characterised in that** the predetermined angle (29) is less than 135 degrees.

13. Bending tool (25) according to claim 12, wherein the predetermined angle (29) is less than 110 degrees.

14. Bending tool (25) according to claim 12 or claim 13, wherein the curved bending surface (24) comprises a longitudinal recess running circumferentially along the bending surface and configured to receive the wand member so as to support a wall of the tubular element during bending for thereby reducing distortion of the wall during bending.

15. Bending tool according to one of claims 12 to 14, wherein the tool is fabricated such that all surface concavities, recesses or inner vertices are formed with a radius of curvature of at least 1mm.

## Patentansprüche

1. Elektrochirurgische Vorrichtung (1), umfassend:
ein biegbares, längliches Stabelement (2);
einen Griff (3) an einem proximalen Ende des Stabelements, wobei der Griff (3) vom Stabelement (2) abnehmbar ist;
einen funktionalen Spitzenabschnitt (4) an einem distalen Ende des Stabelements (2); wobei das Stabelement (2) einen ersten, proximalen Abschnitt (18) aufweist, der gerade bleibt, wenn das Stabelement gebogen wird, einen zweiten, distalen Abschnitt (16), der den funktionalen Spitzenabschnitt (4) umfasst, und einen dritten, biegbaren Abschnitt (17), der sich zwischen dem ersten und dem zweiten Abschnitt (16, 18) befindet, um durch manuelles Biegen mit einem Biegeradius zwischen 10 mm und 50 mm oder vorzugsweise zwischen 20 mm und 50 mm plastisch verformt zu werden, so dass ein Abweichungswinkel (27) zwischen dem ersten und dem dritten Abschnitt von mindestens 20 Grad entsteht;
wobei der erste Abschnitt eine erste Biegefestigkeit und der dritte Abschnitt eine zweite Biegefestigkeit aufweist, die geringer ist als die erste Biegefestigkeit; wobei das Stabelement ein rohrförmiges Element umfasst, das sich im Wesentlichen entlang der Länge des ersten, zweiten und dritten Längenabschnitts erstreckt und aus einem Metall besteht, das im zweiten Längenabschnitt in seinem ungebogenen Zustand einen Kaltverfestigungsexponenten von mehr als 0,4 aufweist;
**dadurch gekennzeichnet, dass**
das Werkzeug eine Blockiereinrichtung (19') zum Verhindern einer Drehung des rohrförmigen Elements relativ zum Griff um eine Längsachse des Stabelements umfasst;
wobei die zweite Biegefestigkeit mindestens 80 % der ersten Biegefestigkeit beträgt, wenn sich das Werkzeug in einer ungebogenen Konfiguration befindet, bevor das Biegen erfolgt; und die zweite Biegefestigkeit mindestens 85 % der ersten Biegefestigkeit beträgt, wenn sich die Vorrichtung in einer gebogenen Konfiguration befindet, nach dem Biegen.

2. Vorrichtung nach Anspruch 1, wobei: das röhrenförmige Element (13) aus einem Metall mit einem Festigkeitskoeffizienten von mehr als 1200 MPa besteht.

3. Vorrichtung nach einem der Ansprüche 1 oder 2, wobei die erste Biegefestigkeit grösser als 200 MPa und die zweite Biegefestigkeit grösser als 180 MPa ist.

4. Vorrichtung nach einem der vorstehenden Ansprüche, wobei das Metall Edelstahl ist.

5. Vorrichtung nach einem der vorstehenden Ansprüche, wobei der erste Längenabschnitt mindestens 80 mm lang ist und der zweite Längenabschnitt mindestens 25 mm lang ist.

6. Vorrichtung nach einem der vorstehenden Ansprüche, wobei das Stabelement (2) so verformbar ist, dass ein Abstand zu einem Mittelpunkt der Biegung (5) vom distalen Ende des Stabelements, gemessen entlang des rohrförmigen Elements (13), weniger als 50 mm beträgt.

7. Vorrichtung nach einem der vorstehenden Ansprüche, wobei die elektrochirurgische Vorrichtung (1) eine monopolare elektrochirurgische Elektrode ist.

8. Vorrichtung nach einem der Ansprüche 1 bis 6, wobei die elektrochirurgische Vorrichtung (1) eine bipolare elektrochirurgische Elektrode ist.

9. Verfahren zur Herstellung einer Vorrichtung gemäss einem der Ansprüche 1 bis 8, **gekennzeichnet durch** einen Verarbeitungsschritt zum Glühen oder Normalisieren des Metalls des zweiten Längenabschnitts, so dass:
- die zweite Biegefestigkeit vor dem Biegen grösser als 80 % der ersten Biegefestigkeit ist;
- die zweite Biegefestigkeit nach dem Biegen grösser als 90 % der ersten Biegefestigkeit ist.

10. Verfahren nach Anspruch 9, wobei der Verarbeitungsschritt ein unterkritisches oder interkritisches Glühen bei einer Temperatur zwischen 500 und 700 Grad oder ein Weichglühen bei einer Temperatur zwischen 700 und 900 Grad umfasst.

11. Verfahren zum Biegen des Stabelements (2) einer elektrochirurgischen Vorrichtung (1) gemäss einem der Ansprüche 1 bis 8, wobei das Verfahren umfasst:
- Bereitstellen eines Biegewerkzeugs (20) mit einer Aussparung (21) zum Sichern des Spitzenabschnitts (4) in verankertem Eingriff während des Biegens, wobei das Biegewerkzeug ferner eine gekrümmte Biegefläche (24) mit einem minimalen Krümmungsradius zwischen 20 mm und 50 mm und einen länglichen Griffabschnitt (25) mit einer Längsachse (25') umfasst;
- Einrasten des Spitzenabschnitts (4) in den verankerten Eingriff mit der Aussparung (21);
- manuelles Ausüben einer Kraft zwischen dem Griffabschnitt (3) und/oder dem Längenabschnitt der elektrochirurgischen Vorrichtung mit einer Hand und dem Griff (25) des Biegewerkzeugs (20) mit der anderen Hand, um den zweiten Längenabschnitt (17) um die Biegefläche (24) zu biegen; **dadurch gekennzeichnet, dass** zu Beginn des Biegens die Längsachsen (16', 25') des Stabelements und des Griffs (25) des Biegewerkzeugs in einem Winkel von weniger als 135 Grad zueinander stehen.

12. Biegewerkzeug (20) zum Biegen des Stabes (2) einer Vorrichtung (1) gemäss einem der Ansprüche 1 bis 8 in einem Verfahren gemäss Anspruch 11, wobei das Biegewerkzeug umfasst:
- einen länglichen Griff (25) mit einer Längsachse;
- eine gekrümmte Biegefläche (24) mit einem minimalen Krümmungsradius zwischen 10 mm und 60 mm, vorzugsweise zwischen 20 mm und 50 mm;
- Eingriffsmittel (21) zum Halten des Spitzenabschnitts (4) des Stabelements (2) in verankertem Eingriff während des Biegens, wobei, wenn der Spitzenabschnitt (4) vor Beginn des Biegens in anfänglichem Eingriff mit den Eingriffsmitteln (21) steht, der zweite Längenabschnitt (17) des Stabelements in Kontakt mit einem Anfangskontaktteil (30) der gekrümmten Biegefläche (24) in Kontakt stehen kann, wobei die Längsachse (18') des Stabes parallel zu einer Tangente (30') an den Anfangskontaktteil (30) der gekrümmten Biegefläche (24) verläuft; wobei sich die besagte Tangente (30') in einem vorbestimmten Winkel (29) zur Längsachse des Griffs des Biegewerkzeugs befindet;
**dadurch gekennzeichnet, dass** der vorbestimmte Winkel (29) weniger als 135 Grad beträgt.

13. Biegewerkzeug (25) nach Anspruch 12, wobei der vorbestimmte Winkel (29) weniger als 110 Grad beträgt.

14. Biegewerkzeug (25) nach Anspruch 12 oder Anspruch 13, wobei die gekrümmte Biegefläche (24) eine Längsausnehmung umfasst, die in Umfangsrichtung entlang der Biegefläche verläuft und so ausgestaltet ist, dass sie das Stabelement aufnimmt, um eine Wand des rohrförmigen Elements während des Biegens zu stützen und dadurch die Verformung der Wand während des Biegens zu verringern.

15. Biegewerkzeug gemäss einem der Ansprüche 12 bis 14, wobei das Werkzeug so hergestellt ist, dass alle Oberflächenvertiefungen, Aussparungen oder inneren Scheitelpunkte mit einem Krümmungsradius von mindestens 1 mm ausgebildet sind.

## Revendications

1. Dispositif électrochirurgical (1) comprenant :
un élément de baguette allongé et flexible (2) ;
une poignée (3) à une extrémité proximale de l'élément de baguette, la poignée (3) étant détachable de l'élément de baguette (2) ;
une partie de pointe fonctionnelle (4) à une extrémité distale de l'élément de baguette (2) ; l'élément de baguette (2) comportant une première partie proximale (18) destinée à rester droite lorsque l'élément de baguette est courbé, une deuxième partie distale (16) comprenant la partie de pointe fonctionnelle (4), et une troisième partie flexible (17), située entre les première et deuxième parties (16, 18), destinée à être déformée plastiquement par flexion manuelle avec un rayon de courbure compris entre 10 mm et 50 mm, ou de préférence entre 20 mm et 50 mm, de manière à fournir un angle de déviation (27) entre les première et troisième parties d'au moins 20 degrés ;
la première partie ayant une première résistance à la flexion et la troisième partie ayant une deuxième résistance à la flexion, inférieure à la première résistance à la flexion ; dans lequel
l'élément de baguette comprend un élément tubulaire s'étendant sensiblement sur la longueur des première, deuxième et troisième parties longitudinales et constitué d'un métal qui, dans la deuxième partie longitudinale, a un exposant d'écrouissage supérieur à 0,4 à l'état non fléchi ; **caractérisé en ce que :**
le dispositif comprend des moyens de blocage (19') pour empêcher une rotation de l'élément tubulaire, par rapport à la poignée, autour d'un axe longitudinal de l'élément de baguette ;
dans lequel la deuxième résistance à la flexion est d'au moins 80 % de la première résistance à la flexion lorsque le dispositif est dans une configuration non fléchie, avant ladite flexion ; et la deuxième résistance à la flexion est d'au moins 85 % de la première résistance à la flexion lorsque le dispositif est dans une configuration fléchie, après ladite flexion.

2. Dispositif selon la revendication 1, dans lequel : l'élément tubulaire (13) est constitué d'un métal ayant un coefficient de résistance supérieur à 1200 MPa.

3. Dispositif selon l'une des revendications 1 ou 2, dans lequel la première résistance à la flexion est supérieure à 200 MPa et la deuxième résistance à la flexion est supérieure à 180 MPa.

4. Dispositif selon l'une des revendications précédentes, dans lequel le métal est de l'acier inoxydable.

5. Dispositif selon l'une des revendications précédentes, dans lequel la première partie longitudinale mesure au moins 80 mm de longueur et la deuxième partie longitudinale mesure au moins 25 mm de longueur.

6. Dispositif selon l'une des revendications précédentes, dans lequel l'élément de baguette (2) est déformable de telle sorte que la distance entre le point médian de ladite courbure (5) et l'extrémité distale de l'élément de baguette, mesurée le long de l'élément tubulaire (13), est inférieure à 50 mm.

7. Dispositif selon l'une des revendications précédentes, dans lequel le dispositif électrochirurgical (1) est une électrode électrochirurgicale monopolaire.

8. Dispositif selon l'une des revendications 1 à 6, dans lequel le dispositif électrochirurgical (1) est une électrode électrochirurgicale bipolaire.

9. Procédé de fabrication d'un dispositif selon l'une des revendications 1 à 8, **caractérisé par** une étape de traitement consistant à recuire ou à normaliser le métal de la deuxième partie de longueur de telle sorte que :
- la deuxième résistance à la flexion avant le fléchissement soit supérieure à 80 % de la première résistance à la flexion ;
- la deuxième résistance à la flexion après le fléchissement soit supérieure à 90 % de la première résistance à la flexion.

10. Procédé selon la revendication 9, dans lequel l'étape de traitement comprend un recuit sous-critique ou intercritique à une température comprise entre 500 et 700 degrés, ou un recuit doux à une température comprise entre 700 et 900 degrés.

11. Procédé de flexion de l'élément de baguette (2) d'un dispositif électrochirurgical (1) selon l'une des revendications 1 à 8, le procédé comprenant :
- la fourniture d'un outil de flexion (20) comprenant un évidement (21) pour fixer la partie de pointe (4) dans un engagement ancré pendant la flexion, l'outil de flexion comprenant en outre une surface de flexion courbée (24) ayant un rayon de courbure minimal compris entre 20 mm et 50 mm, et une partie de poignée allongée (25) ayant un axe longitudinal (25') ;
- engager la partie de pointe (4) dans ledit engagement ancré avec ledit évidement
(21) ; - appliquer manuellement une force entre la partie de poignée (3) et/ou la partie de longueur du dispositif électrochirurgical d'une main et la poignée (25) de l'outil de flexion (20) de l'autre main afin de fléchir la deuxième partie de longueur (17) autour de la surface de flexion (24) ; **caractérisé en ce qu'au** début du fléchissement, les axes longitudinaux (16', 25') de l'élément de baguette et de la poignée (25) de l'outil de fléchissement forment un angle inférieur à 135 degrés l'un par rapport à l'autre.

12. Outil de flexion (20) pour fléchir la baguette (2) d'un dispositif (1) selon l'une des revendications 1 à 8 dans un procédé selon la revendication 11, l'outil de flexion comprenant :
- une poignée allongée (25) ayant un axe longitudinal ;
- une surface de flexion courbée (24) ayant un rayon de courbure minimal compris entre 10 mm et 60 mm, ou de préférence entre 20 mm et 50 mm ;
- des moyens d'engagement (21) pour maintenir la partie de pointe (4) de l'élément de baguette (2) en engagement ancré pendant le cintrage, de sorte que, lorsque la partie de pointe (4) est engagée avec les moyens d'engagement (21) dans un engagement initial avant le début du cintrage, la deuxième partie de longueur (17) de l'élément de baguette peut reposer en contact avec une partie de contact initiale (30) de la surface de cintrage incurvée (24), l'axe longitudinal (18') de la baguette étant parallèle à une tangente (30') à la partie de contact initiale (30) de la surface de flexion courbe (24) ; dans lequel ladite tangente (30') forme un angle prédéterminé (29) avec l'axe longitudinal de la poignée de l'outil de flexion ; **caractérisé en ce que** l'angle prédéterminé (29) est inférieur à 135 degrés.

13. Outil de flexion (25) selon la revendication 12, dans lequel l'angle prédéterminé (29) est inférieur à 110 degrés.

14. Outil de flexion (25) selon la revendication 12 ou la revendication 13, dans lequel la surface de flexion courbe (24) comprend un évidement longitudinal s'étendant circonfé-rentiellement le long de la surface de flexion et configuré pour recevoir l'élément de baguette de manière à supporter une paroi de l'élément tubulaire pendant la flexion afin de réduire ainsi la déformation de la paroi pendant la flexion.

15. Outil de flexion selon l'une des revendications 12 à 14, dans lequel l'outil est fabriqué de telle sorte que toutes les concavités, tous les évidements ou tous les sommets intérieurs de surface sont formés avec un rayon de courbure d'au moins 1 mm.
